(19) Europäisches Patentamt
European Patent Office
Office européen des brevets

(11) **EP 3 366 210 A1**

(12) **EUROPEAN PATENT APPLICATION**
published in accordance with Art. 153(4) EPC

(43) Date of publication:
**29.08.2018 Bulletin 2018/35**

(21) Application number: **16879279.4**

(22) Date of filing: **19.12.2016**

(51) Int Cl.:
*A61B 5/055* (2006.01)   *A61B 5/0488* (2006.01)
*A61B 5/00* (2006.01)   *G01R 33/32* (2006.01)
*G01R 33/54* (2006.01)

(86) International application number:
**PCT/KR2016/014877**

(87) International publication number:
**WO 2017/111412 (29.06.2017 Gazette 2017/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **21.12.2015 KR 20150183012**

(71) Applicant: **Samsung Electronics Co., Ltd.
Suwon-si, Gyeonggi-do 16677 (KR)**

(72) Inventor: **AHN, Ji Hun
Yongin-si
Gyeonggi-do17106 (KR)**

(74) Representative: **Gulde & Partner
Patent- und Rechtsanwaltskanzlei mbB
Wallstraße 58/59
10179 Berlin (DE)**

(54) **MEDICAL IMAGING DEVICE AND MAGNETIC RESONANCE IMAGING DEVICE, AND CONTROL METHOD THEREFOR**

(57) The present invention is directed to providing a medical imaging device and a magnetic resonance imaging device, which are capable of predicting a motion artifact by detecting a motion of a patient by measuring an electromyographic signal of the patient, and a control method thereof. In accordance with an aspect of the present disclosure, a magnetic resonance imaging device comprises: a scanner configured to apply a magnetic field and a radio frequency (RF) signal to a patient and acquire a magnetic resonance signal; a signal measurement unit configured to measure an electromyographic signal of the patient; a controller configured to determine a motion characteristic of the patient based on the measured electromyographic signal and predict whether a motion artifact caused by a motion of the patient will occur on the basis of the determined motion characteristic of the patient; and a data processor configured to store the magnetic resonance signal and reconstruct a magnetic resonance image using the stored magnetic resonance signal.

【Fig. 1】

# Description

[Technical Field]

The present invention relates to a medical imaging device and a magnetic resonance imaging device capable of correcting a motion artifact appearing in a medical image, and a control method thereof.

[Background Art]

Medical imaging devices are devices that portray images of interiors of target objects for diagnostic or therapeutic purposes, and includes devices with a variety of modality, such as magnetic resonance imaging (MRI) devices, radiography devices, mammography devices, positron emission tomography (PET) devices, computed tomography (CT) devices, single photon emission computed tomography (SPECT) devices, optical coherence tomography (OCT) devices, and the like.

No matter which medical imaging device is used, artifacts caused by the motion, that is, motion artifacts, may appear in medical images when the target object moves during medical imaging. In particular, in the case of a modality which takes a relatively long time to take images, such as magnetic resonance imaging or CT imaging, motion artifacts will likely happen and the motion artifacts may be a cause of degrading in the quality of the devices.

[Technical Problem]

The present invention is directed to providing a medical imaging device and a magnetic resonance imaging device, which are capable of predicting a motion artifact by detecting a motion of a patient by measuring an electromyographic signal of the patient, and a control method thereof.

[Technical Solution]

In accordance with an aspect of the present disclosure, a magnetic resonance imaging device comprises: a scanner configured to apply a magnetic field and a radio frequency (RF) signal to a patient and acquire a magnetic resonance signal; a signal measurement unit configured to measure an electromyographic signal of the patient; a controller configured to determine a motion characteristic of the patient based on the measured electromyographic signal and predict whether a motion artifact caused by a motion of the patient will occur on the basis of the determined motion characteristic of the patient; and a data processor configured to store the magnetic resonance signal and reconstruct a magnetic resonance image using the stored magnetic resonance signal.

The controller may control the scanner to reacquire the magnetic resonance signal when the motion artifact is predicted to occur.

The controller may control the scanner to reacquire the magnetic resonance signal at a time of a motion occurrence of the patient when the motion artifact is predicted to occur.

The data processor may delete the magnetic resonance signal acquired at the time of the motion occurrence of the patient among the stored magnetic resonance signal, and reconstructs the magnetic resonance image using the re-acquired magnetic resonance signal.

The data processor may correct the motion artifact by applying a post-processing to the magnetic resonance image when the motion artifact is predicted to occur.

The signal measurement unit may comprise at least one measurement sensor attached to a site associated with a capturing site.

The signal processor may measure the electromyographic signal of the patient from before the magnetic resonance signal starts to be acquired.

The controller may filter the electromyographic signal of the patient measured after the magnetic resonance signal starts to be acquired, using the electromyographic signal of the patient measured before the magnetic resonance signal starts to be acquired

The controller may acquire a motion pattern of the patient by applying a pattern classification to the electromyographic signal of the patient measured after the magnetic resonance signal starts to be acquired; and the motion pattern of the patient may include the motion characteristic of the patient.

The motion pattern of the patient may include information related to at least one of a magnitude of the motion and a type of the motion.

The controller may generate a pattern classifier by applying pattern learning to the electromyographic signal of the patient measured before the magnetic resonance signal starts to be acquired.

The controller may predict that the motion artifact will occur in a case when the motion characteristic of the patient is a preset threshold or more.

When a measuring time of the electromyographic signal is shorter than a reference time, the controller may predict that the motion artifact will occur in a case when the motion characteristic of the patient is a preset threshold or more.

When the measuring time of the electromyographic signal is the reference time or more, the controller may acquire a motion pattern of the patient from the electromyographic signal of the patient by applying a pattern classification to the electromyographic signal of the patient.

In accordance with another aspect of the present disclosure, a medical imaging device comprises: a scanner configured to scan a patient for acquiring a medical image; a signal measurement unit configured to measure electromyographic signals of the patient; and a controller configured to determine a motion characteristic of the patient based on the measured electromyographic signals, and predict whether a motion artifact caused by a motion of the patient will occur on the basis of the determined motion characteristic of the patient.

The signal measurement unit may be configured to measure the electromyographic signal of the patient from before a magnetic resonance signal of the patient starts to be acquired.

The controller may acquire a motion pattern of the patient by applying a pattern classification to the electromyographic signal of the patient measured after the magnetic resonance signal starts to be acquired; and the motion pattern of the patient includes the motion characteristic of the patient.

The controller may predict that the motion artifact will occur in a case when the motion characteristic of the patient is a preset threshold or more.

When a measuring time of the electromyographic signal is shorter than a reference time, the controller may predict that the motion artifact will occur in a case when the motion characteristic of the patient is a preset threshold or more.

When the measuring time of the electromyographic signal is the reference time or more, the controller acquires a motion pattern of the patient from the electromyographic signal of the patient by applying a pattern classification to the electromyographic signal of the patient.

In accordance with another aspect of the present disclosure, a method of controlling a magnetic resonance imaging device, comprises: measuring an electromyographic signal of a patient; performing magnetic resonance imaging with respect to the patient; determining a motion characteristic of the patient based on the electromyographic signal; and predicting whether a motion artifact will occur on the basis of the motion characteristic of the patient.

The method of controlling a magnetic resonance imaging device may further comprise re-acquiring a magnetic resonance signal at a time of a motion occurrence of the patient when the motion artifact is predicted to occur.

The method of controlling a magnetic resonance imaging device may further comprise applying a post-processing to a magnetic resonance image of the patient and correcting the motion artifact when the motion artifact is predicted to occur.

The determining of the motion characteristic of the patient based on the electromyographic signal may include applying a pattern classification to the electromyographic signal of the patient and acquiring a motion pattern of the patient.

The predicting whether a motion artifact will occur on the basis of the motion characteristic of the patient may include predicting that the motion artifact will occur in a case when the motion characteristic of the patient is a preset threshold or more.

[Advantageous Effects]

According to a medical imaging device, a magnetic resonance imaging device, and a control method thereof according to an exemplary embodiment of the present invention, a motion artifact can be predicted by detecting a motion of a patient by measuring an electromyographic signal of the patient.

[Description of Drawings]

FIG. 1 illustrates a control block diagram of a medical imaging device according to an exemplary embodiment of the present invention.
FIG. 2 illustrates a control block diagram of a magnetic resonance imaging device according to an exemplary embodiment of the present invention.
FIGS. 3A and 3B illustrate a schematic view of an exterior of a magnetic resonance imaging device.
FIG. 4 illustrates a view of a space divided by X, Y, and Y axes, in which a target object is disposed.
FIG. 5 illustrates views of a scanner structure and a gradient coil structure.
FIG. 6 illustrates a graph of pulse sequences associated with a gradient coil operation.
FIG. 7 illustrates a diagram of pulse sequences when spin echo pulse sequences are used.
FIG. 8 illustrates a graph of a relationship between a k-space and echo signals acquired several times by changing a magnitude of the z-axis gradient magnetic field.
FIGS. 9 and 10 illustrate a diagram of a structure of the signal measurement unit for measuring the electromyographic signals of the patient.
FIG. 11 illustrates a diagram of one exemplary embodiment of the signal processor.
FIGS. 12 and 13 illustrate a flowchart of a method of controlling a magnetic resonance imaging device according to an exemplary embodiment of the present invention.
FIG. 14 illustrates a flowchart of an example for predicting occurrence of motion artifacts, using a pattern classification, with respect to a magnetic resonance imaging device according to an exemplary embodiment of the present invention.
FIG. 15 illustrates a flowchart of an example for learning patterns, using electromyographic signals acquired from a patient, with respect to a method of controlling a magnetic resonance imaging device according to an exemplary embodiment of the present invention.

[Modes of the Invention]

Hereinafter, a medical imaging device, a magnetic resonance imaging device, and a control method thereof according to an aspect of the present invention will be described in detail with reference to the accompanying drawings.

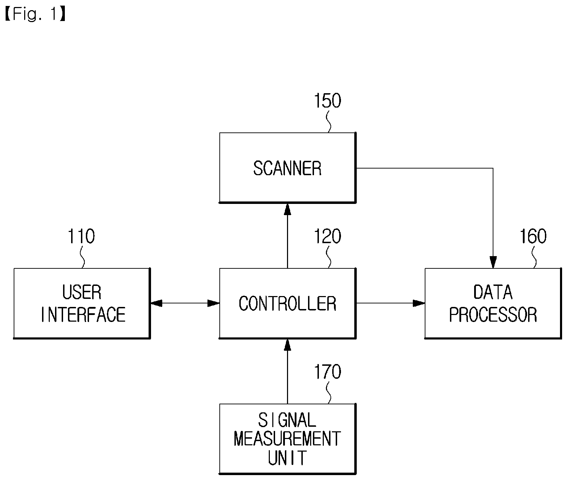

FIG. 1 illustrates a control block diagram of a medical imaging device according to an exemplary embodiment of the present invention.

Referring to FIG. 1, a medical imaging device 100 according to the exemplary embodiment of the present invention includes a scanner 150 for scanning a target object to acquire a medical image, a controller 120 for controlling an operation of the scanner 150, a data processor 160 for processing data acquired by the scanner 150 for scanning the target object to acquire the medical image, a user interface 110 through which the user's control commands for scanning or data processing are received, and a signal measurement unit 170 that measures electromyographic signals of the target object, processes the measured electromyographic signals, and transmits the signals to the controller 120.

An electromyographic signal is an electrical signal generated when a muscle is depolarized, and an analysis of the electromyographic signal enables estimation of a posture or motion of the target object due to changes in the characteristics of the electromyographic signal as the muscle contracts and relaxes.

Therefore, the medical imaging device 100 according to the exemplary embodiment of the present invention may measure the electromyographic signals of the target object using the signal measurement unit 170. When the measured electromyographic signals are transmitted to the controller 120, the controller 120 may determine motion characteristics of the target object based on the electromyographic signals, and predict whether a motion artifact will occur based on the motion characteristics of the target object. In this case, the determination of whether the motion artifact will occur refers to determining likelihood of the motion artifact to occur.

When the controller 120 predicts an occurrence of the motion artifact, the controller 120 may perform an operation for an appropriate response, such as retaking images, performing image post-processing for correcting the motion artifact, or the like.

Hereinafter, a specific operation of each component of the medical imaging device 100 will be described. In the following exemplary embodiments, an example of a specific modality will be described for convenience, that is, an example of a magnetic resonance imaging device in which it takes a long time to take images and is highly likely to generate a motion artifact will be described.

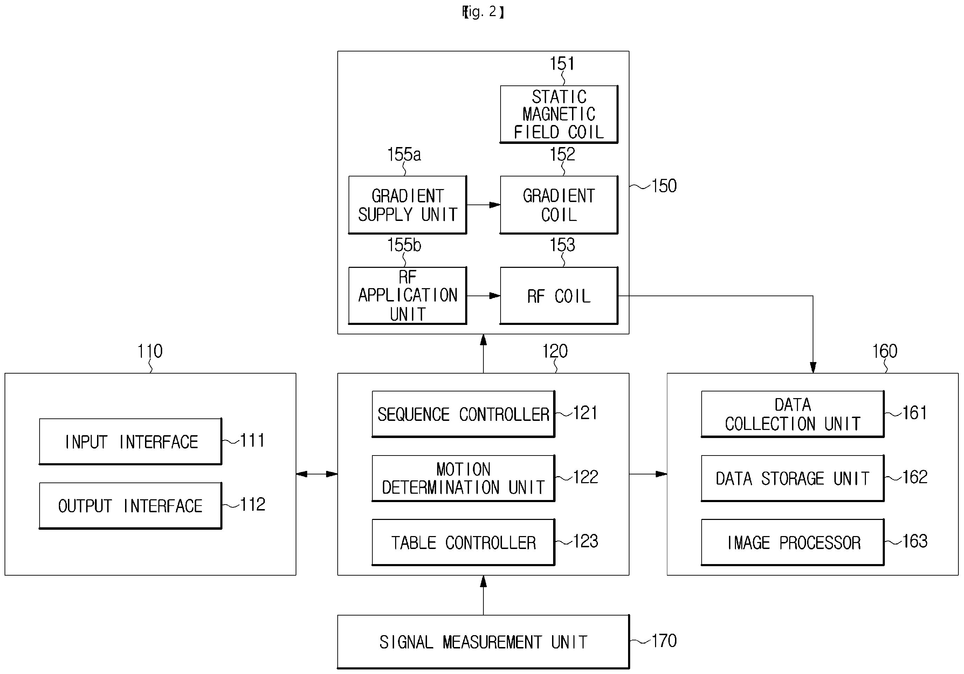

FIG. 2 illustrates a control block diagram of a magnetic resonance imaging device according to an exemplary embodiment of the present invention.

Referring FIG. 2, a scanner 150 forms a magnetic field and receives a magnetic resonance signal generated from a target object. To this end, the scanner 150 includes a static magnetic field coil 151 for forming a static magnetic field, a gradient coil 152 for forming a gradient magnetic field, and a radio frequency (RF) coil 153 that applies an RF pulse to the target object to excite atomic nuclei and receives an echo signal from the atomic nuclei.

In addition, the scanner 150 may further include a gradient supply unit 155a, which applies a gradient current for generating gradient magnetic field in the gradient coil 152, and an RF application unit 155b for transmitting an RF signal to the RF coil 153. For instance, the gradient supply unit 155a may be implemented as a circuit including a gradient amplifier, and the RF application unit 155b may include a pre-amplifier and a modulation circuit for modulating a high frequency signal into a pulse type signal.

The controller 120 may include a sequence controller 121, a motion determination unit 122, and a table controller 123.

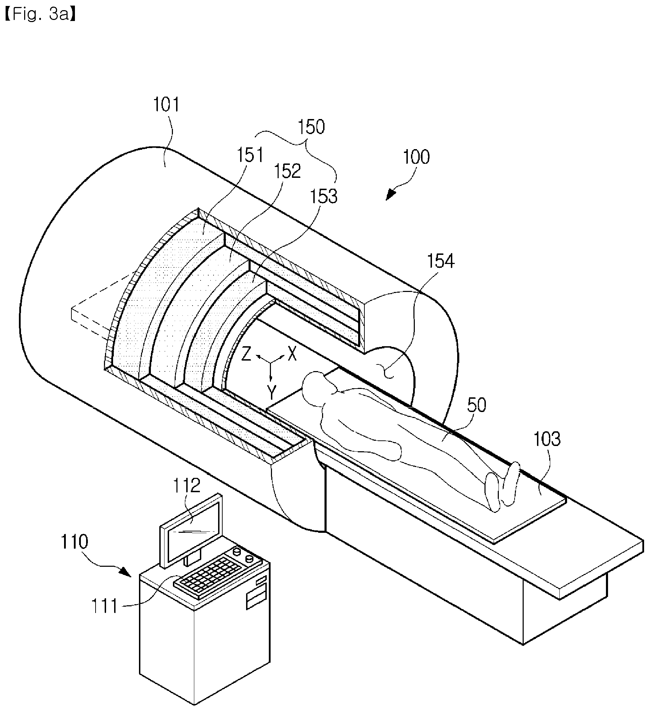

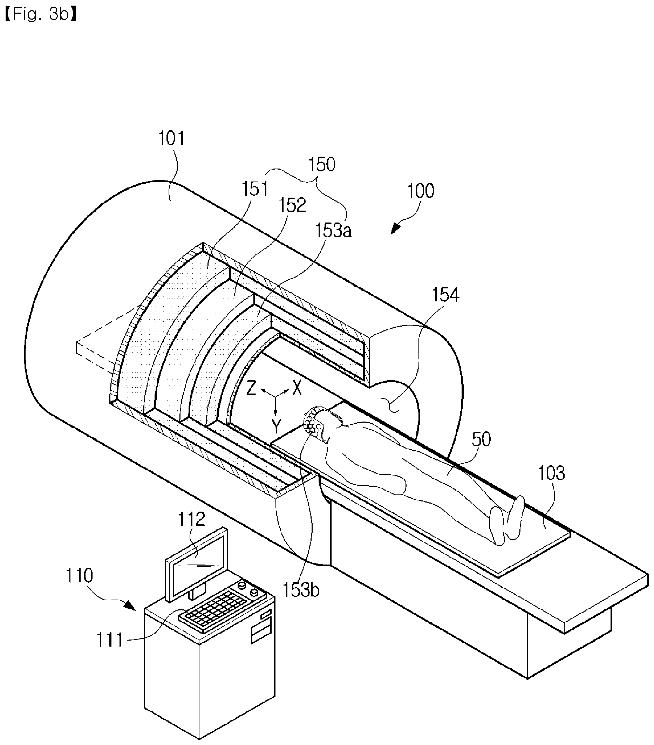

The sequence controller 121 may control an RF pulse applied to the target object 50 and gradient magnetic field formed in a bore 154 (see FIG. 3) by controlling the gradient coil 152 and the RF coil 153 through the gradient supply unit 155a and the RF application unit 155b.

Thus, the sequence controller 121 is connected to the gradient supply unit 155a to control timing, shape, and the like of a gradient waveform, and connected to the RF application unit 155b to control timing, magnitude, and shape of RF pulse.

The motion determination unit 122 may determine the motion of the target object based on the electromyographic signals provided by the signal measurement unit 170. For example, a pattern classification may be used, or a threshold indicating a motion occurrence may be set. A detailed description of the motion determination will be described below.

The controller 120 may include a program for performing the above-described operations and below-described operations, a memory for temporarily storing data required to execute the program, and a processor for executing the program stored in the memory. For example, the controller 120 may be implemented by a chip such as a micro controller unit (MCU), a central processor (CPU), or the like.

The sequence controller 121 and the motion determination unit 122 may share a processor or memory, or use separate processors and memories. Further, the controller 120 may share a processor or memory, or a single computer with some or all components of the data processor 160 or signal measurement unit 170.

The RF coil 153 is connected to the data processor 160, and the data processor 160 may include a data collection unit 161 for receiving magnetic resonance signals acquired by the RF coil 153, a data storage unit 162 for storing k-space data, and an image processor 163 for restoring a magnetic resonance image using the k-space data.

The data collection unit 161 may include a receiver for receiving the magnetic resonance signal acquired by the RF coil 153, a pre-amplifier for amplifying the magnetic resonance signal, a phase detector for receiving the magnetic resonance signal from the pre-amplifier and detecting a phase thereof, and an analog-to-digital (A/D) converter for converting an analog signal acquired by detecting the phase into a digital signal. The data collection unit 161 transmits a digitally converted magnetic resonance signal to the data storage unit 162.

A mathematical space for storing magnetic resonance data is formed in the data storage unit 162, and this mathematical space refers to the k-space. As an example, the k-space may be a two-dimensional Fourier space.

When the k-space data is completed by filling the k-space of the data storage unit 162 with magnetic resonance data, the image processor 163 applies various image restoration techniques to generate a magnetic resonance image, for example, the k-space data may be inversely Fourier-transformed to restore an image.

In addition, the magnetic resonance imaging device 100 may include a user interface 110 containing an input interface 111 and an output interface 112. Control commands for overall operations of the magnetic resonance imaging device 100 may be input by a user through the input interface 111. In particular, when commands on photographing techniques of magnetic resonance images or pulse sequences are received from the user, the sequence controller 121 may control the pulse sequences according to the received commands.

The output interface 112 may display various types of information about control of the magnetic resonance imaging device 100 and magnetic resonance images generated by the image processor 163.

The input interface 111 may include at least one of various input devices such as a keyboard, a mouse, a trackball, a keypad, a touchpad, and the like, and the output interface 112 may include at least one of various output devices such as a liquid crystal display (LCD), a light-emitting diode (LED), an organic light-emitting diode (OLED), a plasma display panel (PDP), a cathode ray tube (CRT), and the like.

In addition, the input interface 111 and the output interface 112 may be implemented in a touch screen form such that a touch panel is disposed on a front surface of a display.

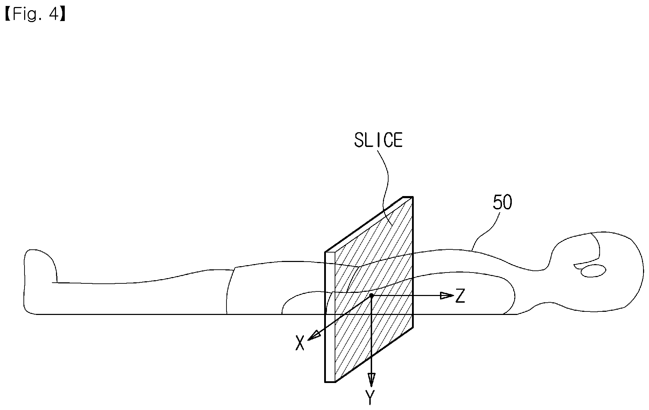

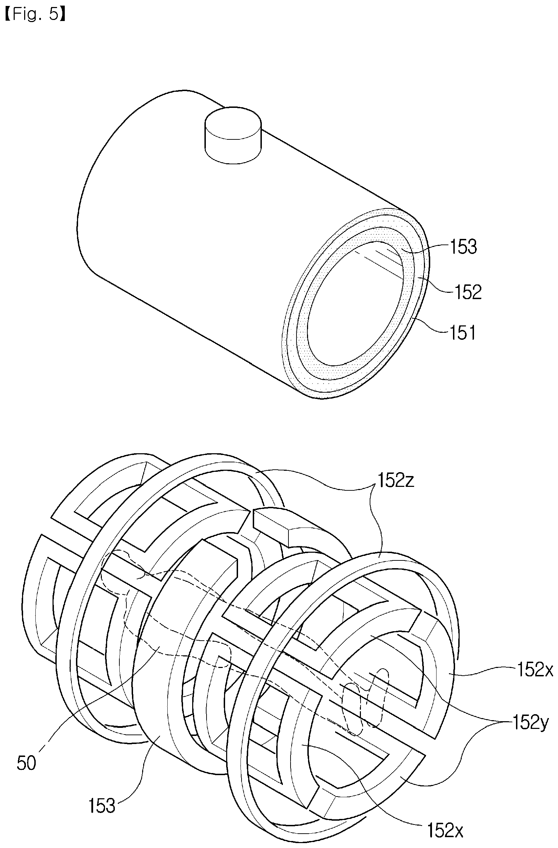

FIGS. 3A and 3B illustrate a schematic view of an exterior of a magnetic resonance imaging device, FIG. 4 illustrates a view of a space divided by X, Y, and Y axes, in which a target object is disposed, and FIG. 5 illustrates views of a scanner structure and a gradient coil structure.

Hereinafter, specific operations of the magnetic resonance imaging device 100 will be described with reference also to FIG. 2 described above.

Referring to FIGS. 3A and 3B, a static magnetic field coil 151, a gradient coil 152, and an RF coil 153 may be built in a gantry 101 having a hollow-cylindrical shape.

A table 103 transfers a target object 50 lying thereon to the bore 154, and a table controller 123 controls and moves the table 103 to a position desired by a user when the user manipulates the input interface 111 to input a command on a position of the table 103.

Meanwhile, the target object may be just an object of which an magnetic resonance image can be acquired by the magnetic resonance imaging device 100, and there is no restriction on a type thereof. However, the target object will refer to a patient in the following exemplary embodiments.

For instance, the table controller 123 may move the table 103 to locate a capturing site of the patient at an isocenter of a magnetic field formed in the bore 154. The farther the site is from the isocenter of the magnetic field formed in the bore 154, the lower the homogeneity of the magnetic field. Thus, the magnetic resonance data should be acquired as close as possible to the isocenter to minimize image distortion. The isocenter represents a center of the magnetic field formed in the bore, which indicates a point at which all of an x-axis gradient magnetic field Gz, a y-axis gradient magnetic field Gy, and a z-axis gradient magnetic field Gz are zero.

The user interface 110, as shown in FIGS. 3A and 3B, may be included in a workstation or host device provided separately from the gantry 101. Further, some or all components of the controller 120, the data processor 160, the signal measurement unit 170 may also be included in the workstation or host device.

A transmitting coil and receiving coil of the RF coil 153 may be built in the gantry 101 as shown in FIG. 3A, or a transmitting coil 153a may be built in the gantry 101 and a receiving coil 153b may be directly attached to the capturing site of the patient 50. For example, when the capturing site is the patient's head, the receiving coil 153b may be implemented in a form of a helmet.

A surface coil, a volume coil, and an array coil may be used as an example of the receiving coil 153b.

The static magnetic field coil 151 includes a coil for generating a static magnetic field in the bore, and refers to a main magnet. The main magnet may be implemented as a superconducting magnet. In this case, the static magnetic field coil 151 may include a superconducting magnet.

The static magnetic field coil 151 may be implemented in a form in which a coil is wrapped around the bore 154, and the static magnetic field having a constant magnitude is formed by the static magnetic field coil 151. A direction of the static magnetic field is generally parallel to a longitudinal axis of the gantry 101.

When the static magnetic field is formed in the bore 154, atoms constituting the patient, particularly, nuclei of hydrogen atoms, are oriented in a direction of the static magnetic field and perform a precession around the direction of the static magnetic field. A speed of the procession of the nuclei may be expressed in terms of a precessional frequency, which refers to Larmor frequency that may be expressed by the following equation.

## [Equation 1]

$$\omega = \gamma B_0$$

In this case, co is a Larmor frequency, $\gamma$ is a proportional constant, and $B_0$ is strength of an external magnetic field. The proportional constant varies depending on a type of nucleus, a unit for the strength of the external magnetic field is Tesla (T) or Gauss (G), and a unit for the processional frequency is Hertz (Hz).

For example, since a hydrogen proton has a precessional frequency of 42.58 MHz in the external magnetic field of IT and the hydrogen accounts for the largest proportion of atoms that make up a human body, the magnetic resonance signal is acquired using a precession of the hydrogen proton in a magnetic resonance image.

As shown in FIG. 4, under a premise that a longitudinal axis of the patient 50 is parallel to the longitudinal axis of the gantry 101, a z-axis may be set as an axis parallel to the longitudinal axis of the patient 50 from his or her head to his or her foot, that is, an axis parallel to a direction of a static magnetic field. An x-axis may be set as an axis parallel to a lateral axis of the patient 50, and a y-axis may be set as an axis parallel to a up/down direction of a space.

When the longitudinal axis of the patient 50 is oriented to be parallel to the direction of the static magnetic field, a cross-section image of a lateral cross-section of the patient 50 may be acquired and a slice having a constant thickness may be chosen to acquire the cross-section image.

Gradient magnetic fields for all of the x, y, and z-axes are required to acquire three-dimensional spatial information. Thus, the gradient coil 152 may include three pairs of gradient coils corresponding to the x, y, and z-axes.

As shown in FIG. 5, a z-axis gradient coil 152z is generally implemented as a pair of ring-type coils, and a y-axis gradient coil 152y is disposed above and below the patient 50. An x-axis gradient coil 152x is disposed at a left and right side of the patient 50.

As described below, the z-axis gradient coil 152z is used to select the slice, and the y-axis gradient coil 152y is used for phase encoding, and the x-axis gradient 152z is used for frequency encoding.

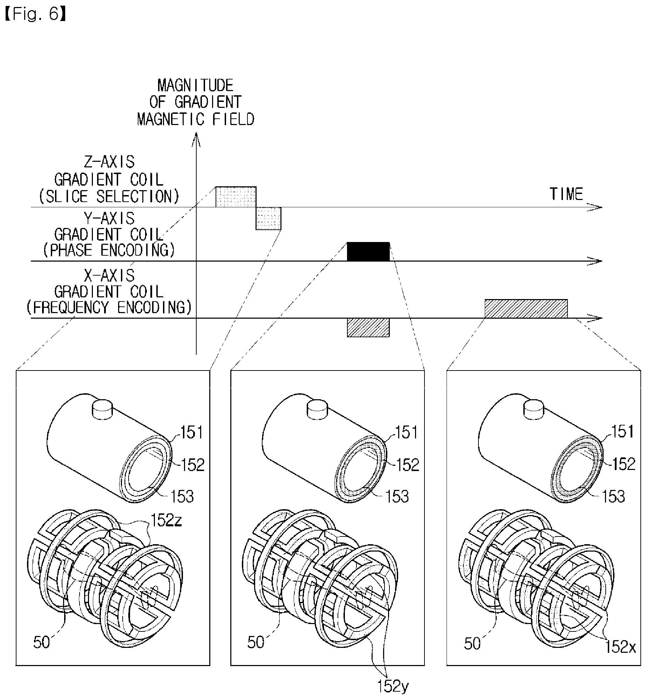

FIG. 6 illustrates a graph of pulse sequences associated with a gradient coil operation.

When direct currents having opposite polarities flow in two z-axis gradient coils 152z in opposing directions, a change of a magnetic field occurs in the z-axis to form a gradient magnetic field.

When the magnetic field is formed by having a current flowing through the z-axis gradient coil 152z during a certain time, a resonance frequency is increased or decreased depending on a magnitude of the magnetic field. Also, when the RF coil 153 generates an RF pulse corresponding to a certain position, protons only in a cross-section corresponding to the certain position cause resonance. Thus, the z-axis gradient coil 152z is used for slice selection. Further, the larger a slope of the gradient magnetic field formed in the z-axis direction is, the thinner a thickness of the slice may be selected.

When the slice is selected with the gradient magnetic field formed by the z-axis gradient coil 152z, all spins constituting the slice have a same frequency and a same phase, and may not be distinguishable from each other.

In this case, when the gradient magnetic field in the y-axis direction is formed by the y-axis gradient coil 152y, the gradient magnetic field leads to a phase shift such that rows constituting the slice have a different phase from each other.

In other words, when a y-axis gradient magnetic field is formed, phases for spins of a row, to which a large gradient magnetic field is applied, change to ones with a high frequency, and phases for spins of a row, to which a small gradient magnetic field is applied, change to ones with a low frequency. When the y-axis gradient magnetic field disappears, each row of the selected slice comes to have a different phase due to occurrence of a phase shift, and thereby the rows may be distinguishable. According to this principle, the gradient magnetic field generated by the y-axis gradient coil 152y may be used for phase encoding.

A slice is selected with the gradient magnetic field formed by the z-axis gradient coil 152z, and the rows constituting the selected slice are distinguished from each other with the gradient magnetic field formed by the y-axis gradient coil 152y. However, all the spins constituting a single row have a same frequency and a same phase, and thus may not be distinguishable from each other.

In this case, when the gradient magnetic field is formed in the x-axis direction by the x-axis gradient coil 152x, the x-axis gradient magnetic field allows the spins constituting each row to have different frequencies from each other and distinguishes each spin from other. In this manner, the gradient magnetic field formed by the x-axis gradient coil 152x is used for frequency encoding.

As described above, the gradient magnetic field formed by the z-axis gradient coil, the y-axis gradient coil, and the x-axis gradient coil spatially encodes a spatial position of each spin by slice selection, phase encoding, and frequency encoding.

The gradient coil 152 is connected to the gradient supply unit 155a, and the gradient supply unit 155a generates the gradient magnetic field by applying a gradient waveform, that is, a current pulse to the gradient coil 152 according to a control signal transmitted from the sequence controller 121. Therefore, the gradient supply unit 155a refers to a gradient power source, and may include three driving circuits that correspond to the three gradient coils 152x, 152y, and 152z constituting the gradient coil 152.

The nuclei oriented by an external magnetic field as described above perform precession with a Larmore frequency, and a magnetization vector sum of several nuclei may be denoted by a single net magnetization M.

A z-axis component of the net magnetization may not be measured while only $M_{XY}$ may be detected. Thus, in order to acquire the magnetic resonance signal, the net magnetization should be present on an x-y plane by exciting the nuclei. The RF pulse tuned with the Larmor frequency of the nuclei should be applied to excite the nuclei.

As an example of a pulse sequence used to acquire the magnetic resonance signal from the nuclei, there are gradient echo pulse sequence, spin echo pulse sequence, and the like. Hereinafter, an example of the spin echo pulse sequence will be described in detail.

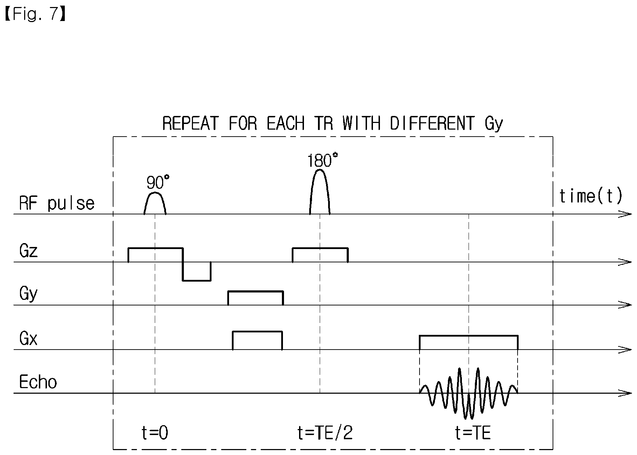

FIG. 7 illustrates a diagram of pulse sequences when spin echo pulse sequences are used.

Referring to FIG. 7, when an RF pulse is applied by the RF coil 153, an RF pulse for exciting the nuclei (hereinafter, referred to as an excited RF) is first applied. When the excited RF pulse is applied, non-uniformity of a magnetic field or an interaction between spins causes dephasing, and at this point, a sharply dropping free induction decay (FID) signal occurs.

Therefore, when a refocusing RF pulse for recollecting dephased spins is applied to acquire a stable signal, strong transverse magnetization occurs in the nuclei, the FID signals are gathered again such that a stable echo signal, that is, a magnetic resonance signal occurs. This refers to a spin echo pulse sequence, and a time period between the application of the excited RF and the occurrence of the magnetic resonance signal refers to an echo time (TE).

When a time period between the application of the excited RF pulse and the application of the refocusing RF pulse is $\Delta t$, a magnetic resonance signal is generated in the case that the time period $\Delta t$ has elapsed after the application of the re-focusing RF pulse. Thus, a relation of TE = 2$\Delta t$ is established.

How much the proton has been flipped may be represented as an angle moved from the axis that was located prior to flipping, and it may be represented as a 90° RF, a 180° RF, and the like depending on a degree of flipping. In the spin-echo pulse sequence, the excited RF is mainly set as a 90° RF and the refocusing RF is set as a 180° RF.

Referring to FIG. 7, when a z-axis gradient magnetic field is applied simultaneously with a 90° RF pulse, resonance occurs only at a position corresponding to a certain cross-section. When a y-axis gradient magnetic field is applied and then blocked to give position information to a signal, a phase difference occurs between the protons.

When a 180° RF pulse is applied to acquire a spin echo signal, the spins dephased by non-uniformity of the magnetic field or a chemical shift are re-magnetized.

When the x-axis gradient magnetic field Gx is applied after the 180° RF pulse is applied, a re-phase by the 180° RF pulse is added to acquire a maximum echo signal.

The generated echo signal includes position information on the x and and y axes. Therefore, signals acquired by the y-axis gradient magnet field and x-axis gradient magnetic field fill a k-space, which is a two-dimensional space consisting of a kx-axis and a ky-axis.

Meanwhile, the echo signal may be acquired several times by changing a magnitude of the y-axis gradient magnetic field to acquire one cross-sectional image, a time interval between a time of applying the 90° RF to acquire an echo signal and a time of applying the 90° RF to acquire the next echo signal refers to a repetition time (TR).

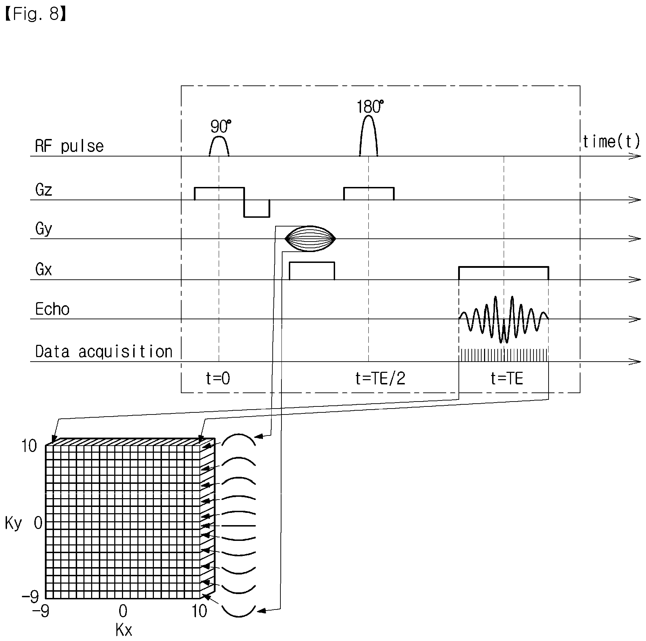

FIG. 8 illustrates a graph of a relationship between a k-space and echo signals acquired several times by changing a magnitude of the z-axis gradient magnetic field.

As described above, a k-space is formed in the data storage unit 162, and a k-space data, which is data stored in the k-space, is stored in the data storage unit 162. The k-space is a sort of a mathematical space for performing a Fourier transformation, which consists of a kx-axis and ky-axis as shown in FIG. 8, and the kx-axis is a frequency direction and the ky-axis is a phase direction.

For example, when echo signals are acquired for 20 different y-axis gradient magnetic fields as shown in FIG. 8, the ky-axis may consist of 20 horizontal lines, that is, 20 ky lines, and an echo signal acquired for each gradient magnetic field fills a single ky line. Thus, when all the echo signals are acquired for the 20 different y-axis gradient magnetic fields, all the 20 ky lines are filled to complete a single k-space data. When one k-space data is restored, one magnetic resonance image may be acquired.

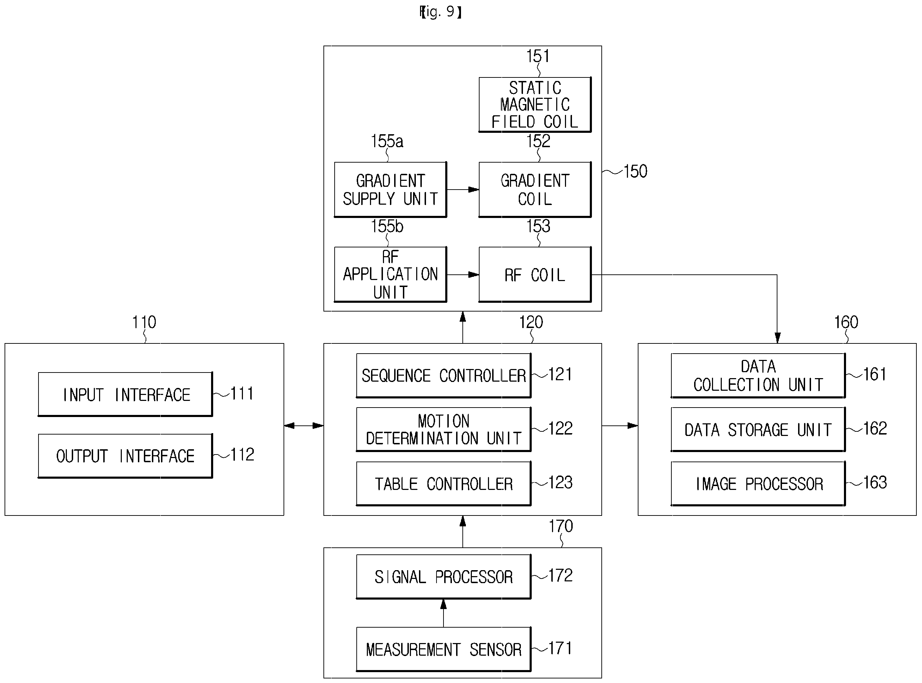

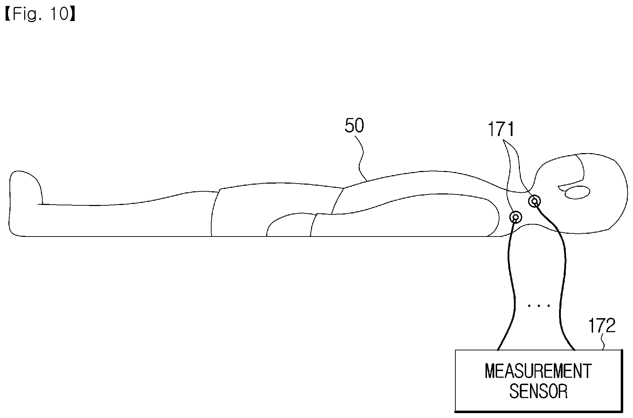

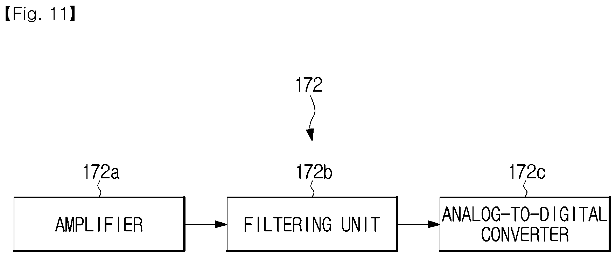

FIGS. 9 and 10 illustrate a diagram of a structure of the signal measurement unit for measuring the electromyographic signals of the patient, and FIG. 11 illustrates a diagram of one exemplary embodiment of the signal processor.

Referring FIGS. 9 and 10, the signal measurement unit 170 may include a measurement sensor 171 attached to the patient 50 and a signal processor 172 that processes signals measured by the measurement sensor 171 and converts the signals into a form usable for motion determination.

The measurement sensor 171 may be mounted on a site associated with a capturing site. The site associated with the capturing site represents a site in which a motion artifact may appear on a magnetic resonance image when the site is moved.

For example, the measurement sensor 171 may be attached to an erector spinae muscle, a quadratus lumborum, and an internal oblique of the patient 50 when a back or waist of the patient 50 is photographed, and the measurement sensor 171 may be attached to an upper trapezoid muscle of the patient 50 when a neck of the patient 50 is photographed.

In addition, when a heart of the patient 50 is photographed, the measurement sensor 171 may be attached to a chest muscle of the patient 50.

Also, when a head of the patient 50 is photographed, the measurement sensor 171 may be attached to a part of a neck muscle of the patient 50, as shown in FIG. 10. The measurement sensor 171 may be provided with a plurality of measurement sensors as needed.

Therefore, a user may measure electromyographic signals by attaching an appropriate number of the measurement sensors 171 to appropriate positions in which a motion of the site to be photographed may be detected.

Referring to an example of FIG. 11, the signal processor 172 may include an amplifier 172a for receiving and amplifying the electromyographic signals measured by the measurement sensor 171, and a filtering unit 172b for filtering the amplified signals, and an analog-to-digital converter 172c for converting the filtered signals to digital signals.

The amplifier 172a may be selected from among various amplifiers such as a pre-amplifier, a differential amplifier, an instrument amplifier, and the like.

The filtering unit 172b may be selected from among various filters such as a notch filter, a high pass filter, a low pass filter, and the like.

The measurement of the electromyographic signal may be made from before a scan (magnetic resonance imaging) starts. The measured electromyographic signal measured before the scan is made may be a background signal, and the measured electromyographic signal measured after the scan is made becomes a main signal used for the motion determination. The filtering unit 172b tunes a bias by first filtering the main signal using the background signal.

In addition, the filtering unit 172b may perform second filtering in a frequency domain after first filtering. To this end, a frequency conversion may be performed using a fast Fourier transform (FFT).

The filtered electromyographic signal may be converted into a digital signal through the analog-to-digital converter 172c and input to the motion determination unit 122.

The signal processor 172 described above may include at least one processor and memory, and may be implemented by a chip such as an MCU or a CPU. The signal processor 172 may be implemented as a separate module or chip from the controller 120 or share a module or chip with the controller 120.

The motion determination unit 122 may determine motion characteristics using the input electromyographic signal, and predict whether motion artifacts are generated on the basis of the motion characteristics. In this case, the input electromyographic signal may be used with no change or used after being converted into a value such as a root mean square (RMS), a median frequency (MDF), a mean frequency (MNF), or the like.

As an example for determining the motion characteristics, a pattern classification may be used. The motion determination unit 122 may generate a pattern classifier by applying one of various models used for pattern recognition and performing pattern learning, and output a pattern corresponding to the motion of the patient, that is, a motion pattern when the electromyographic signal is input to the pattern classifier.

The pattern classifier may be generated in advance prior to when the magnetic resonance imaging starts. For example, a motion database may be constructed based on a relationship between the generated electromyographic signal and an actual motion by collecting electromyographic signals from a plurality of patients. The motion determination unit 122 may perform the pattern learning using data stored in the motion database and generate the pattern classifier.

It is also possible to perform pattern learning using the electromyographic signals collected from the patient and generate a pattern classifier. In this case, using the electromyographic signals collected from before the magnetic resonance imaging, the pattern learning may be performed and the pattern classifier may be generated. Further, even after the magnetic resonance imaging starts, the electromyographic signals may be continuously measured and used to update the pattern classifier.

There is no restriction on methods or models used for the pattern learning and classification. Any of a variety of pattern learning and pattern classification models based on machine learning may be applied.

For example, a covariance method and a learning vector quantization may be applied. The covariance method is a method of estimating autoregressive (AR) parameters, the learning vector quantization is one of methods for supervised learning clustering in pattern recognition, which is a network for clustering input patterns into multiple output layers. The learning vector quantization is characterized in determining a similarity between an input pattern and a reference pattern, and newly updating the reference pattern.

When an electromyographic signal is input to the pattern classifier, a motion pattern corresponding to the input electromyographic signal is output. Each motion pattern may represent a motion characteristic such as a magnitude or type of a motion. Here, the type of motion may indicate a posture change of the patient. For example, when the measurement sensor 171 is attached to a neck muscle of the patient to detect a head motion of the patient, the posture change of the patient such as whether the patient turns his or her head to the left or right, whether the patient bows forward or backward, and the like may be indicated. Further, a magnitude of the motion may be represented as a degree to which the patient's posture has changed.

The motion determination unit 122 may predict whether the motion artifact occurs based on a motion pattern of the patient. Even though the patient's motion occurs, a magnitude or type thereof may not affect the occurrence of the motion artifact.

For example, though in principle, the patient should not breathe while a magnetic resonance image is being taken, the patient may not follow this principle, and it is practically not possible to hold the breath continuously during the taking when a sequence of the imaging takes a long time. Since the data acquired while the patient is breathing constitutes a single k-space, a motion artifact may occur in a restored magnetic resonance image. As a method of reducing the motion artifact, there are prospective gating or retrospective gating methods in which an image is restored using data on the same breathing state based on a characteristic of periodic repetition of respiration.

In the case of applying the gating methods, it is considered that the motion caused by the breathing of the patient generates no motion artifact, and a motion other than a motion caused by the breathing may be determined as the motion generating the motion artifact.

Thus, in the case of performing a scan influenced by motions of muscles around a chest, for example, where a capturing site is a heart, the motion determination unit 122 may form a breath pattern by performing pattern learning during the inhalation and exhalation of the patient before the scan starts.

When the scan starts, the signal measuring unit 171 acquires an electromyographic signal and transmits the electromyographic signal to the motion determination unit 122. When the motion pattern indicated by the electromyographic signal is not a pattern due to breathing, the motion determination unit 122 may predict that a motion artifact will occur.

Therefore, the motion determination unit 122 may predict whether a motion artifact will occur based on information about a relationship between the motion pattern of the patient and the occurrence of the motion artifact, and transmit a result to the sequence controller 121 or the data processor 160. For example, the information on the relationship between the motion pattern of the patient and the occurrence of the motion artifact may be information on whether the motion artifact will occur for each motion pattern or information matched with the likelihood of occurrence of the motion artifact.

As an example, when the motion determination unit 122 predicts the occurrence of the motion artifact, the motion determination unit 122 may transmit a motion artifact prediction signal to all of the sequence controller 121 and the data processor 160. The sequence controller 121 receiving the motion artifact prediction signal may control the scanner 150 to re-acquire a magnetic resonance signal at a time of the motion occurrence of the patient. Further, the data storage unit 162 may delete, among the stored data, the magnetic resonance signal acquired when the patient's motion occurs, and the image processor 163 may reconstruct an image using the re-acquired magnetic resonance signal.

As another example, when the motion determination unit 122 predicts the occurrence of the motion artifact, the motion determination unit 122 may transmit the motion pattern of the patient to the data processor 160. The motion pattern of the patient may be stored in a memory, and the image processor 163 may perform a post-processing for correcting the motion artifact on the basis of the stored motion pattern.

Specifically, since post-processing algorithms for correcting the occurred motion artifact, post-processing parameters, and the like may be changed according to the magnitude and type of the motion, the image processor 163 may perform the post-processing for correcting the motion artifact with reference to the motion pattern of the patient.

In addition, the re-acquiring the magnetic resonance signal and the correction by the post-processing may be selectively performed on the basis of the motion pattern. For example, the magnetic resonance signal is re-acquired when the magnitude of the motion is a preset reference value or more, and the correction by post-processing may be performed when the magnitude of the motion is smaller than the preset reference value. Further, it may be efficient to re-acquire the magnetic resonance signal for each motion pattern, or to perform the correction by post-processing. Therefore, it is also possible that each motion pattern may be mapped to one of re-acquiring the magnetic resonance signal and correcting by the post-processing.

As another example for determining the motion, a threshold value may be preset and the electromyographic signal may be compared with the threshold value. When the electromyographic signal indicates the threshold value or more, motion artifacts may be predicted to occur and an appropriate action may be taken. Operations after the occurrence of the motion artifact is predicted are the same as in the above-described example.

In addition, it is also possible to select between comparing with the threshold value according to reliability of the pattern classifier or performing a pattern classification. For example, when a measurement time of the electromyographic signal is less than a reference time, a method of comparing the electromyographic signal with the preset threshold value may be selected because the reliability of the pattern classifier is not high due to insufficient number of data used for pattern learning. When the measurement time of the electromyographic signal is the reference time or longer, a method of performing the pattern classification may be chosen under a premise that the pattern classifier is reliable.

Hereinafter, an exemplary embodiment of a magnetic resonance imaging device according to one aspect of the present invention will be described. The magnetic resonance imaging device according to the above-described exemplary embodiment may be used for a method of controlling a magnetic resonance imaging device according to the exemplary embodiment. Thus, the above description with reference to FIGS. 1 to 11 is also applicable to the method of controlling a magnetic resonance imaging apparatus, even when there is no mention thereof.

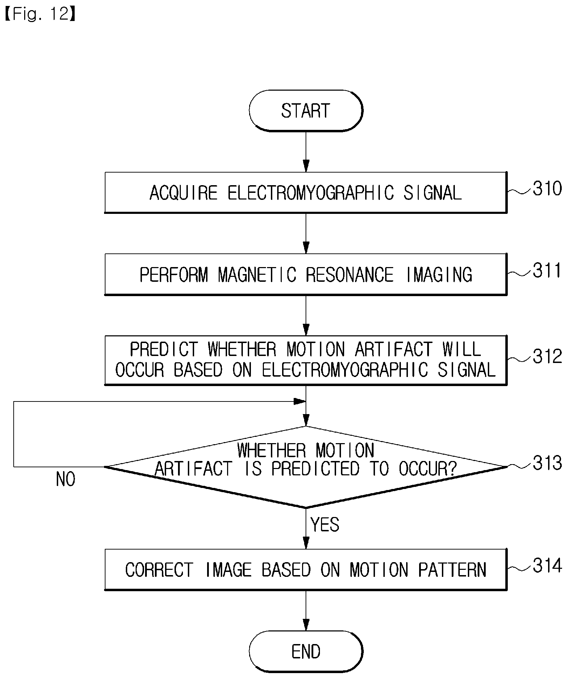

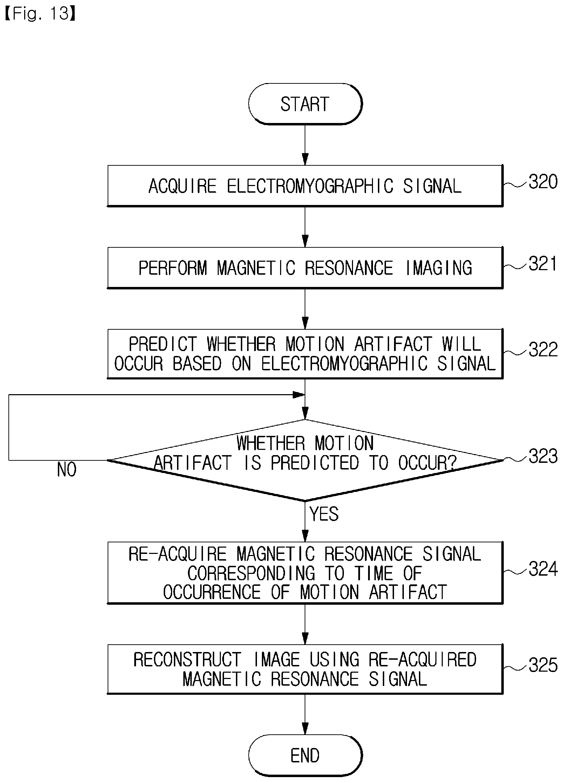

FIGS. 12 and 13 illustrate a flowchart of a method of controlling a magnetic resonance imaging device according to an exemplary embodiment of the present invention.

Referring to FIG. 12, at least one measurement sensor 171 is attached to a site associated with a capturing site of the patient to acquire an electromyographic signal (310). The site associated with the capturing site of the patient refers to a site at which motion artifacts may appear on a magnetic resonance image thereof when moved. For example, muscles used to move the capturing site may be the site associated with the capturing site. When the capturing site is a head, neck muscles may be the site associated with the capturing site. When the capturing site is a heart, chest muscles may be the site associated with the capturing site.

Magnetic resonance imaging is performed (311). In other words, a scan of the capturing site may be initiated. In this case, the scan may include both of a main scan and a pre-scan made before the main scan. The main scan may be performed by the sequence controller 121 controlling the scanner 150 to apply a pulse sequence for acquiring a magnetic resonance signal of the capturing site. Meanwhile, the electromyographic signal may be acquired in real time even while the magnetic resonance imaging is performed.

While the electromyographic signal is acquired in real time and the magnetic resonance imaging is performed, the motion determination unit 122 may predict whether the motion artifact will occur on the basis of the electromyographic signal. For example, the motion pattern of the patient may be acquired on the basis of the electromyographic signal, and the motion pattern of the patient may represent a magnitude or type of the motion. Also, whether the motion artifact will occur may be predicted based on the motion pattern of the patient.

When the occurrence of the motion artifact is predicted (YES in operation 313), the magnetic resonance image is corrected based on the motion pattern of the patient (314). Specifically, when the motion determination unit 122 predicts the occurrence of the motion artifact, the motion determination unit 122 may transmit the motion pattern of the patient to the data processor 160. The motion pattern of the patient is stored in a memory, and the image processor 163 may perform a post-processing for correcting the motion artifact on the basis of the stored motion pattern. Specifically, since post-algorithms for correcting the occurred motion artifact, post-processing parameters, and the like may be changed according to the magnitude or type of the motion, the image processor 163 may perform the post-processing for correcting the motion artifact with reference to the motion pattern of the patient.

Meanwhile, when the motion artifact is predicted to occur, a corresponding action is not limited to the image correction. Hereinafter, the exemplary embodiment will be described with reference to FIG. 13.

Referring FIG. 13, since operations of acquiring the electromyographic signal (320), performing the magnetic resonance imaging (321), and determining whether the motion artifact will occur (322) are the same as those in the example of FIG. 12 described above, thus will be omitted.

In this exemplary embodiment, when the motion artifact is predicted to occur (YES in operation 323), a magnetic resonance signal corresponding to an occurrence time of the motion artifact (324) may be re-acquired. In this case, the magnetic resonance signal corresponding to an occurrence time of the motion artifact may be a magnetic resonance signal acquired when the motion causing the motion artifact occurs. The data storage unit 162 may delete, among the stored data, the magnetic resonance signal acquired at the time of the motion occurrence of the patient, and the image processor 163 may reconstruct the image using the re-acquired magnetic resonance signal.

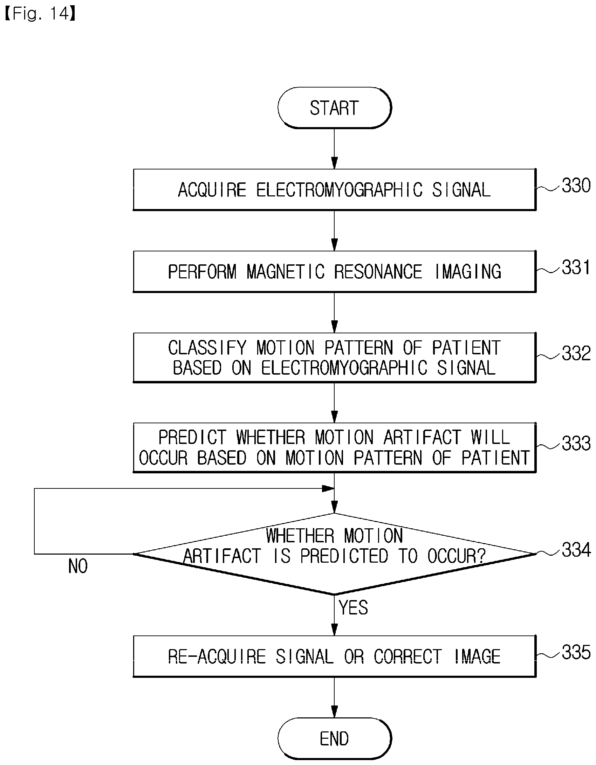

FIG. 14 illustrates a flowchart of an example for predicting occurrence of motion artifacts, using a pattern classification, with respect to a magnetic resonance imaging device according to an exemplary embodiment of the present invention.

Referring to FIG. 14, at least one measurement sensor 171 is attached to a site associated with a capturing site of the patient to acquire an electromyographic signal (330).

Magnetic resonance imaging is performed (331). The electromyographic signal may be acquired in real time even while the magnetic resonance imaging is performed.

While the electromyographic signal is acquired and the magnetic resonance imaging is performed, the motion determination unit 122 may classify motion patterns of the patient in real time on the basis of the electromyographic signal (332). The motion determination unit 122 may generate a pattern classifier by implementing pattern learning by applying one of various models used for pattern recognition, and a pattern corresponding to the motion of the patient, that is, the motion pattern may be output when the electromyographic signal is input in the pattern classifier.

The pattern classifier may be generated in advance prior to starting magnetic resonance imaging, and generated after pattern learning is performed using the electromyographic signal collected from the patient.

Whether the motion artifact will occur may be predicted based on the motion pattern of the patient (333). The motion determination unit 122 may predict whether the motion artifact will occur on the basis of information about a relationship between the motion pattern of the patient and the occurrence of the motion artifact. As an example, the information on the relationship between the motion pattern of the patient and the occurrence of the motion artifact may be information on whether the motion artifact will occur for each motion pattern or information matched with the likelihood of occurrence of the motion artifact.

When the motion artifact is predicted to occur (YES in operation 334), the motion artifact may be removed by re-acquiring the magnetic resonance signal or correcting the magnetic resonance image (335).

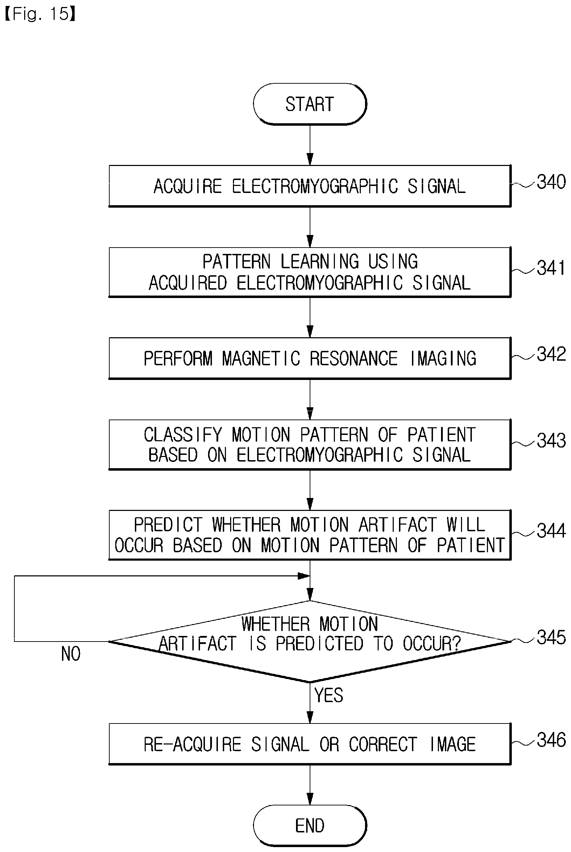

FIG. 15 illustrates a flowchart of an example for learning patterns, using electromyographic signals acquired from a patient, with respect to a method of controlling a magnetic resonance imaging device according to an exemplary embodiment of the present invention.

Referring to FIG. 15, at least one measurement sensor 171 is attached to a site associated with a capturing site of the patient and then an electromyographic signal is acquired (340). As described above, the electromyographic signal may be acquired in advance prior to when the magnetic resonance scan starts, and continues to be acquired after the magnetic resonance scan starts.

The pattern learning is performed using the electromyographic signals collected before the magnetic resonance imaging (341), and a pattern classifier is generated. In this case, since the pattern learning is performed using the electromyographic signals of the issued patient, reliability of the pattern classification result may be improved.

Magnetic resonance imaging is performed (342). The motion determination unit 122 may classify the motion pattern of the patient in real time based on the electromyographic signal even while the electromyographic signal is acquired and the magnetic resonance imaging is performed (343).

Whether the motion artifact will occur may be predicted based on the motion pattern of the patient (344), and when the motion artifact is predicted to occur (YES in operation 345), the motion artifact may be removed by re-acquiring the magnetic resonance signal or correcting the magnetic resonance image (346).

The motion pattern of the patient may be stored in a memory to be constructed as a database. Therefore, when the patient is subsequently photographed again for the magnetic resonance image, the pattern classifier constructed as the database may be called up to update and reuse.

In addition, information about the motion pattern of the patient may also be stored in the memory to construct a database of a motion habit of the patient, the database of the motion habit may be reflected in post-processing of the image, selection of the pulse sequence, or update of the pattern classifier.

Meanwhile, as another example for determining the motion, a threshold value may be preset and the electromyographic signal may be compared with the threshold value. When the electromyographic signal indicates the threshold value or more, motion artifacts may be predicted to occur and an appropriate action may be taken. Operations after the occurrence of the motion artifact is predicted are the same as in the above-described example.

In addition, it is also possible to select between comparing with the threshold value according to the reliability of the pattern classifier or performing a pattern classification. For example, when a measurement time of the electromyographic signal is less than a reference time, a method of comparing the electromyographic signal with a preset threshold value may be selected because the reliability of the pattern classifier is not high due to insufficient number of data used for pattern learning. When the measurement time of the electromyographic signal is the reference time or longer, a method of performing the pattern classification may be chosen under the premise that the pattern classifier is reliable.

Although the above-described exemplary embodiments have been described with the example of the magnetic resonance imaging device, the above-described exemplary embodiments may be applied to medical imaging devices other than the magnetic resonance imaging device, such as positron emission tomography (PET) devices, computed tomography (CT) devices, and the like.

Also, the foregoing description is merely illustrative of technical idea of the present invention, and various modifications, alterations, and substitutions are possible without departing from essential characteristics of the present invention. Therefore, the exemplary embodiments and accompanying drawings described above are intended to illustrate but is not to limit the technical idea, and a scope of technical spirit of the present invention is not limited by the exemplary embodiments and accompanying drawings. The scope of the present invention is to be construed in accordance with the following claims, and all technical ideas within the scope of the same should be construed as being included in the scope of the present invention.

Further, terms used herein are used to illustrate the exemplary embodiments and are not intended to limit and/or to restrict the present invention. Singular forms "a," "an," and "the" include plural referents unless the context clearly dictates otherwise. In the specification, terms "comprises," "have," or the like are used to specify that there are stated features, numbers, steps, ooperations, elements, parts, or combinations thereof, but do not preclude presence or addition of one or more of a different feature, a different number, a different step, a different operation, a different element, a different component, or a different combination thereof.

In addition, the terms "unit," "-er," and the like used in the entire specification may represent a unit for performing at least one of functions or operations.

**Claims**

1.  A magnetic resonance imaging device comprising:

    a scanner configured to apply a magnetic field and a radio frequency (RF) signal to a patient and acquire a magnetic resonance signal;
    a signal measurement unit configured to measure an electromyographic signal of the patient;
    a controller configured to determine a motion characteristic of the patient based on the measured electromyographic signal and predict whether a motion artifact caused by a motion of the patient will occur on the basis of the determined motion characteristic of the patient; and
    a data processor configured to store the magnetic resonance signal and reconstruct a magnetic resonance image using the stored magnetic resonance signal.

2.  The magnetic resonance imaging device of claim 1, wherein the controller controls the scanner to re-acquire the magnetic resonance signal when the motion artifact is predicted to occur.

3.  The magnetic resonance imaging device of claim 1, wherein the controller controls the scanner to re-acquire the magnetic resonance signal at a time of a motion occurrence of the patient when the motion artifact is predicted to occur.

4.  The magnetic resonance imaging device of claim 3, wherein the data processor deletes the magnetic resonance signal acquired at the time of the motion occurrence of the patient among the stored magnetic resonance signal, and reconstructs the magnetic resonance image using the re-acquired magnetic resonance signal.

5.  The magnetic resonance imaging device of claim 1, wherein the data processor corrects the motion artifact by applying a post-processing to the magnetic resonance image when the motion artifact is predicted to occur.

6.  The magnetic resonance imaging device of claim 1, wherein the signal processor measures the electromyographic signal of the patient from before the magnetic resonance signal starts to be acquired.

7.  The magnetic resonance imaging device of claim 6, wherein the controller filters the electromyographic signal of the patient measured after the magnetic resonance signal starts to be acquired, using the electromyographic signal of the patient measured before the magnetic resonance signal starts to be acquired

8.  The magnetic resonance imaging device of claim 6, wherein the controller acquires a motion pattern of the patient by applying a pattern classification to the electromyographic signal of the patient measured after the magnetic resonance signal starts to be acquired; and
    the motion pattern of the patient includes the motion characteristic of the patient.

9.  The magnetic resonance imaging device of claim 6, wherein the controller generates a pattern classifier by applying pattern learning to the electromyographic signal of the patient measured before the magnetic resonance signal starts to be acquired.

10. The magnetic resonance imaging device of claim 1, wherein, when a measuring time of the electromyographic signal is shorter than a reference time, the controller predicts that the motion artifact will occur in a case when the motion characteristic of the patient is a preset threshold or more.

11. The magnetic resonance imaging device of claim 10, wherein, when the measuring time of the electromyographic signal is the reference time or more, the controller acquires a motion pattern of the patient from the electromyographic signal of the patient by applying a pattern classification to the electromyographic signal of the patient.

12. A method of controlling a magnetic resonance imaging device, comprising:

    measuring an electromyographic signal of a patient;
    performing magnetic resonance imaging with respect to the patient;
    determining a motion characteristic of the patient based on the electromyographic signal; and
    predicting whether a motion artifact will occur on the basis of the motion characteristic of the patient.

13. The method of controlling a magnetic resonance imaging device of claim 12, further comprising re-acquiring a magnetic resonance signal at a time of a motion occurrence of the patient when the motion artifact is predicted to occur.

14. The method of controlling a magnetic resonance imaging device of claim 12, further comprising applying a post-processing to a magnetic resonance image of the patient and correcting the motion artifact when the motion artifact is predicted to occur.

15. The method of controlling a magnetic resonance imaging device of claim 12, wherein the determining of the motion characteristic of the patient based on the electromyographic signal includes applying a pattern classification to the electromyographic signal of the patient and acquiring a motion pattern of the patient.

【Fig. 1】

[Fig. 2]

EP 3 366 210 A1

【Fig. 3a】

【Fig. 3b】

【Fig. 4】

【Fig. 5】

【Fig. 6】

MAGNITUDE
OF GRADIENT
MAGNETIC FIELD

Z-AXIS
GRADIENT COIL
(SLICE SELECTION)

Y-AXIS
GRADIENT COIL
(PHASE ENCODING)

X-AXIS
GRADIENT COIL
(FREQUENCY ENCODING)

TIME

151
152
153
152z
50

151
152
153
50
152y

151
152
153
50
152x

【Fig. 7】

REPEAT FOR EACH TR WITH DIFFERENT Gy

RF pulse — 90° ... 180° ... time(t)

Gz

Gy

Gx

Echo

t=0 ... t=TE/2 ... t=TE

【Fig. 8】

[Fig. 9]

STATIC MAGNETIC FIELD COIL — 151

GRADIENT SUPPLY UNIT — 155a

GRADIENT COIL — 152

RF APPLICATION UNIT — 155b

RF COIL — 153

150

INPUT INTERFACE — 111

OUTPUT INTERFACE — 112

110

SEQUENCE CONTROLLER — 121

MOTION DETERMINATION UNIT — 122

TABLE CONTROLLER — 123

120

DATA COLLECTION UNIT — 161

DATA STORAGE UNIT — 162

IMAGE PROCESSOR — 163

160

SIGNAL PROCESSOR — 172

MEASUREMENT SENSOR — 171

170

【Fig. 10】

【Fig. 11】

172

172a

AMPLIFIER

172b

FILTERING UNIT

172c

ANALOG-TO-DIGITAL CONVERTER

【Fig. 12】

START

ACQUIRE ELECTROMYOGRAPHIC SIGNAL ~310

PERFORM MAGNETIC RESONANCE IMAGING ~311

PREDICT WHETHER MOTION ARTIFACT WILL OCCUR BASED ON ELECTROMYOGRAPHIC SIGNAL ~312

WHETHER MOTION ARTIFACT IS PREDICTED TO OCCUR? ~313

NO

YES

CORRECT IMAGE BASED ON MOTION PATTERN ~314

END

【Fig. 13】

```
        ┌─────────────┐
        │    START    │
        └──────┬──────┘
               │
               ▼
   ┌─────────────────────────────────┐
   │ ACQUIRE ELECTROMYOGRAPHIC SIGNAL │──～320
   └─────────────────┬───────────────┘
                     │
                     ▼
   ┌─────────────────────────────────┐
   │ PERFORM MAGNETIC RESONANCE IMAGING │──～321
   └─────────────────┬───────────────┘
                     │
                     ▼
   ┌─────────────────────────────────┐
   │ PREDICT WHETHER MOTION ARTIFACT WILL │──～322
   │ OCCUR BASED ON ELECTROMYOGRAPHIC SIGNAL │
   └─────────────────┬───────────────┘
                     │
          ┌──────────┘
          │          ▼
          │   ╱────────────────────╲
       NO │  ╱   WHETHER MOTION     ╲──～323
          └─╲ ARTIFACT IS PREDICTED TO OCCUR? ╱
             ╲────────────────────╱
                     │ YES
                     ▼
   ┌─────────────────────────────────┐
   │ RE-ACQUIRE MAGNETIC RESONANCE SIGNAL │──～324
   │     CORRESPONDING TO TIME OF      │
   │    OCCURRENCE OF MOTION ARTIFACT  │
   └─────────────────┬───────────────┘
                     │
                     ▼
   ┌─────────────────────────────────┐
   │ RECONSTRUCT IMAGE USING RE-ACQUIRED │──～325
   │     MAGNETIC RESONANCE SIGNAL    │
   └─────────────────┬───────────────┘
                     │
                     ▼
               ┌───────────┐
               │    END    │
               └───────────┘
```

【Fig. 14】

START

ACQUIRE ELECTROMYOGRAPHIC SIGNAL ～330

PERFORM MAGNETIC RESONANCE IMAGING ～331

CLASSIFY MOTION PATTERN OF PATIENT
BASED ON ELECTROMYOGRAPHIC SIGNAL ～332

PREDICT WHETHER MOTION ARTIFACT WILL
OCCUR BASED ON MOTION PATTERN OF PATIENT ～333

WHETHER MOTION
ARTIFACT IS PREDICTED TO OCCUR? ～334

NO

YES

RE-ACQUIRE SIGNAL OR CORRECT IMAGE ～335

END

【Fig. 15】

```
                    ┌─────────┐
                    │  START  │
                    └────┬────┘
                         │
                         ▼
    ┌──────────────────────────────────────────┐
    │    ACQUIRE ELECTROMYOGRAPHIC SIGNAL       │──~340
    └──────────────────┬───────────────────────┘
                       │
                       ▼
    ┌──────────────────────────────────────────┐
    │         PATTERN LEARNING USING            │──~341
    │    ACQUIRED ELECTROMYOGRAPHIC SIGNAL      │
    └──────────────────┬───────────────────────┘
                       │
                       ▼
    ┌──────────────────────────────────────────┐
    │    PERFORM MAGNETIC RESONANCE IMAGING     │──~342
    └──────────────────┬───────────────────────┘
                       │
                       ▼
    ┌──────────────────────────────────────────┐
    │   CLASSIFY MOTION PATTERN OF PATIENT      │──~343
    │    BASED ON ELECTROMYOGRAPHIC SIGNAL      │
    └──────────────────┬───────────────────────┘
                       │
                       ▼
    ┌──────────────────────────────────────────┐
    │   PREDICT WHETHER MOTION ARTIFACT WILL    │──~344
    │  OCCUR BASED ON MOTION PATTERN OF PATIENT │
    └──────────────────┬───────────────────────┘
                       │
         ┌─────────────┤
         │             ▼
         │          ╱─────────────────────────╲
         │  NO     ╱    WHETHER MOTION          ╲──~345
         └────────⟨  ARTIFACT IS PREDICTED TO OCCUR? ⟩
                   ╲                            ╱
                    ╲──────────────────────────╱
                                │ YES
                                ▼
    ┌──────────────────────────────────────────┐
    │      RE-ACQUIRE SIGNAL OR CORRECT IMAGE   │──~346
    └──────────────────┬───────────────────────┘
                       │
                       ▼
                 ┌─────────┐
                 │   END   │
                 └─────────┘
```

## INTERNATIONAL SEARCH REPORT

| International application No. |
|---|
| **PCT/KR2016/014877** |

**A. CLASSIFICATION OF SUBJECT MATTER**

*A61B 5/055(2006.01)i, A61B 5/0488(2006.01)i, A61B 5/00(2006.01)i, G01R 33/32(2006.01)i, G01R 33/54(2006.01)i*

According to International Patent Classification (IPC) or to both national classification and IPC

**B. FIELDS SEARCHED**

Minimum documentation searched (classification system followed by classification symbols)
A61B 5/055; A61B 5/04; G06K 9/36; G01N 33/48; A61B 5/11; A61B 5/00; A61B 5/103; A61B 5/0488; G01R 33/32; G01R 33/54

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: MRI, electromyogram, movement, artifact, prediction

**C. DOCUMENTS CONSIDERED TO BE RELEVANT**

| Category* | Citation of document, with indication, where appropriate, of the relevant passages | Relevant to claim No. |
|---|---|---|
| X | WO 2014-147519 A1 (KONINKLIJKE PHILIPS N.V.) 25 September 2014 See paragraphs [09]-[19] and figure 1. | 1-3,6-13,15 |
| Y | | 4,5,14 |
| Y | JP 07-194575 A (HITACHI MEDICAL CORP.) 01 August 1995 See abstract, paragraph [13] and figure 2. | 4 |
| Y | KR 10-2015-0042760 A (SAMSUNG ELECTRONICS CO., LTD.) 21 April 2015 See paragraph [182] and figures 3-7. | 5,14 |
| A | KR 10-2014-0049916 A (SAMSUNG ELECTRONICS CO., LTD.) 28 April 2014 See claims 1-27 and figures 1-19. | 1-15 |
| A | US 2012-0301005 A1 (FUDERER) 29 November 2012 See claim 1 and figures 1-4. | 1-15 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 APRIL 2017 (12.04.2017) | **12 APRIL 2017 (12.04.2017)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (January 2015)

INTERNATIONAL SEARCH REPORT
Information on patent family members

International application No.

**PCT/KR2016/014877**

| Patent document cited in search report | Publication date | Patent family member | Publication date |
|---|---|---|---|
| WO 2014-147519 A1 | 25/09/2014 | CN 105142503 A<br>EP 2976000 A1<br>JP 2016-514508 A<br>US 2016-0000383 A1 | 09/12/2015<br>27/01/2016<br>23/05/2016<br>07/01/2016 |
| JP 07-194575 A | 01/08/1995 | NONE | |
| KR 10-2015-0042760 A | 21/04/2015 | CN 105491946 A<br>EP 3010412 A1<br>KR 10-1716421 B1<br>US 2014-0378816 A1<br>US 2015-0208981 A1<br>WO 2014-204277 A1 | 13/04/2016<br>27/04/2016<br>14/03/2017<br>25/12/2014<br>30/07/2015<br>24/12/2014 |
| KR 10-2014-0049916 A | 28/04/2014 | CN 103767706 A<br>CN 103767706 B<br>EP 2721997 A1<br>EP 2721997 B1<br>JP 05-774645 B2<br>JP 2014-083435 A<br>JP 2015-198958 A<br>US 2014-0111199 A1<br>WO 2014-061896 A1 | 07/05/2014<br>04/05/2016<br>23/04/2014<br>14/09/2016<br>09/09/2015<br>12/05/2014<br>12/11/2015<br>24/04/2014<br>24/04/2014 |
| US 2012-0301005 A1 | 29/11/2012 | CN 101490577 A<br>CN 101490577 B<br>EP 2044454 A1<br>JP 2009-543648 A<br>JP 5243421 B2<br>RU 2009105486 A<br>RU 2434238 C2<br>US 2009-0257629 A1<br>US 8244011 B2<br>US 8913811 B2<br>WO 2008-010126 A1 | 22/07/2009<br>03/10/2012<br>08/04/2009<br>10/12/2009<br>24/07/2013<br>27/08/2010<br>20/11/2011<br>15/10/2009<br>14/08/2012<br>16/12/2014<br>24/01/2008 |

Form PCT/ISA/210 (patent family annex) (January 2015)